# EUROPÄISCHE PATENTSCHRIFT

(11) **EP 3 055 671 B1**
(45) Veröffentlichungstag und Bekanntmachung des Hinweises auf die Patenterteilung: **17.01.2018**
(21) Anmeldenummer: 14795542.1
(22) Anmeldetag: 15.09.2014
(51) Int. Cl.: G01N 21/3577, G01N 21/47, G01N 21/85, G01N 33/28, G01M 13/04, G01N 21/01, G01N 21/359, G01N 21/3554, F16C 33/66, F16C 19/52

(54) **SENSOREINHEIT ZUM BESTIMMEN VON EIGENSCHAFTEN EINES SCHMIERMITTELS SOWIE MASCHINENELEMENT UND MASCHINENANORDNUNG**
SENSOR UNIT FOR DETERMINING PROPERTIES OF A LUBRICANT AND MACHINE ELEMENT AND MACHINE ASSEMBLY
ENSEMBLE CAPTEUR SERVANT À DÉTERMINER DES PROPRIÉTÉS D'UN LUBRIFIANT, ÉLÉMENT DE MACHINE ET AGENCEMENT DE MACHINE

(30) Priorität: 10.10.2013 DE 102013220457
(43) Veröffentlichungstag der Anmeldung: 17.08.2016
(73) Patentinhaber: Schaeffler Technologies AG & Co. KG, 91074 Herzogenaurach (DE)
(72) Erfinder: KRAM, Martin, 97447 Gerolzhofen (DE); DRESCHER, Thomas, 96193 Wachenroth (DE); NEUSCHAEFER-RUBE, Stephan, 91074 Herzogenaurach (DE); BILL, Andreas, 90513 Zirndorf (DE)
(86) Internationale Anmeldenummer: PCT/DE2014/200473
(87) Internationale Veröffentlichungsnummer: WO 2015/051792

(56) Entgegenhaltungen:
- WO-A1-2014/202066
- DE-A1-102007 042 254
- DE-A1-102008 019 500
- DE-B3-102013 110 320
- US-B1- 6 331 704
- US-B1- 6 582 661
- "Zustandsüberwachung von Schmierfetten in Wälzlagern", , September 2010 (2010-09), Seiten 1-6, XP055156784, Gefunden im Internet: URL:http://www.schaeffler.com/remotemedien /media/_shared_media/08_media_library/01_p ublications/schaeffler_2/reprint/downloads _16/rp_ssd_21_de_de.pdf [gefunden am 2014-12-04]

## Beschreibung

Die vorliegende Erfindung betrifft eine Sensoreinheit zum Bestimmen von Eigenschaften eines Schmiermittels; beispielsweise eines Schmierfettes in einem Wälzlager. Im Weiteren betrifft die Erfindung ein Maschinenelement mit der erfindungsgemäßen Sensoreinheit und eine Maschinenanordnung mit dem erfindungsgemäßen Maschinenelement.

Die DE 10 2007 042 254 A1 zeigt eine Messvorrichtung zur Analyse des Schmiermittels in einem Lager. Diese Messvorrichtung umfasst einen Sender zum Aussenden infraroter Strahlung, welche auf einen Probenbereich gerichtet ist und von der Probe zurück auf einen Empfänger trifft. Der Probenbereich ist im Inneren des Lagers angeordnet. Von dem Empfänger wird insbesondere IR-Strahlung im Bereich der Kombinationsmethoden der C-H-Schwingungen erfasst und spektral analysiert. Hierdurch kann das Schmiermittel während des Betriebes permanent überwacht werden und es kann ein Alarm ausgegeben werden, wenn das Schmiermittel zu wechseln ist. Bei der gezeigten Ausführungsform befinden sich der Sender und der Empfänger mit einer Einheit zur Signalaufbereitung in einem gemeinsamen Gehäuse. In diesem Gehäuse befinden sich keine Komponenten zur Signalauswertung, durch welche beispielsweise ermittelte Eigenschaften des Schmiermittels unmittelbar als digitales Signal ausgegeben werden können.

Die DE 10 2010 031 919 A1 lehrt eine Messsonde für einen Sensor zur Analyse eines Mediums mittels Infrarotspektroskopie. Mit dieser Messsonde kann beispielsweise das Schmiermittel eines Lagers analysiert werden. Die Messsonde umfasst einen Infrarot-Sender und einen Infrarot-Detektor sowie einen Referenzdetektor. Der Infrarot-Detektor und der Referenzdetektor sind auf zueinander beabstandeten Flächen angeordnet. Vom Infrarot-Sender trifft IR-Strahlung auf das Medium und wird zurück auf den Infrarot-Detektor geworfen. Weiterhin trifft IR-Strahlung vom Infrarot-Sender auf eine Spiegelfläche und wird zurück auf den Referenzdetektor geworfen. Der Infrarot-Sender und der Referenzdetektor sind auf einem Träger angeordnet. Die Spiegelfläche und der Infrarot-Detektor sind auf entgegengesetzten Seiten eines Brückenelementes ausgebildet, wobei der Träger und das Brückenelement beabstandet sind. Es ergibt sich ein dreidimensionaler Aufbau der Komponenten.

Aus der DE 10 2009 037 424 A1 ist eine Lageranordnung bekannt, bei welcher ein Schmiermittelsensor über einen Transmitter Informationen über den Zustand des Lagers zu einer zentralen Empfangsstation sendet.

Die DE 10 2010 015 084 A1 zeigt ein Sensorteil für einen Infrarot-Sensor zur beabstandeten Anordnung eines Infrarot-Senders, eines Infrarot-Detektors, einer Referenzquelle und eines Referenzdetektors.

Aus dem Produktinformationsblatt "FAG GreaseCheck" der Schaeffler Technologies AG und Co. KG, August 2013 ist ein Schmierfettsensor bekannt, welcher insbesondere zur Schmierfettüberwachung in Wälzlagern vorgesehen ist. Der Schmierfettsensor umfasst einen zylinderförmigen Sensorkopf, welcher unmittelbar in das Wälzlager ragt. Der Sensorkopf ist über ein Kabel mit einer Auswerteelektronik verbunden, die in einem größeren Gehäuse beabstandet zum Sensorkopf angeordnet ist. Vom Sensorkopf werden analoge Signale zur Auswertelektronik gesendet. Daher darf die Länge des Kabels nicht mehr als ein Meter betragen. Die Auswerteelektronik in dem zusätzlichen Gehäuse erschwert den Einsatz des Schmierfettsensors.

Aus dem Produktinformationsblatt "Zustandsüberwachung von Schmierfetten in Wälzlagern" der Schaeffler Technologies GmbH und Co. KG, September 2010 ist ebenfalls ein Schmierfettsensor bekannt, welcher insbesondere zur Schmierfettüberwachung in Wälzlagern vorgesehen ist. Der Schmierfettsensor umfasst einen zylinderförmigen Sensorkopf, welcher unmittelbar in das Wälzlager ragt. Der Sensorkopf umfasst eine analoge Auswerteelektronik. Vom Sensorkopf werden ausgewertete analoge Signale, oder durch setzen einer Triggerschwelle, digitale Sensorsignale an einem Digitalausgang generiert. Die Aufgabe der vorliegenden Erfindung besteht ausgehend von dem bekannten Schmierfettsensor darin, die Einsetzbarkeit des Schmierfettsensors zu erleichtern.

Die genannte Aufgabe wird gelöst durch eine Sensoreinheit gemäß dem beigefügten Anspruch 1 sowie durch ein Maschinenelement gemäß dem beigefügten nebengeordneten Anspruch 8 und durch eine Maschinenanordnung gemäß dem beigefügten nebengeordneten Anspruch 9. Die erfindungsgemäße Sensoreinheit dient der Bestimmung von Eigenschaften eines Schmiermittels. Das Schmiermittel befindet sich bevorzugt in Verwendung, insbesondere zum Zwecke der Schmierung von beweglichen Komponenten eines Maschinenelementes. Hierzu ist die Sensoreinheit bevorzugt zur Montage an dem Maschinenelement bzw. in dem Maschinenelement ausgebildet. Die Sensoreinheit ist dabei bevorzugt zur Überwachung des Schmiermittels im Maschinenelement ausgebildet.

Die erfindungsgemäße Sensoreinheit umfasst zunächst ein Sensorgehäuse, welches eine Außenhülle der Sensoreinheit bildet. Das Sensorgehäuse weist ein Fenster auf, welches auf das Schmiermittel ausrichtbar ist und bevorzugt in das Schmiermittel eintauchbar ist. Das Sensorgehäuse ist insbesondere zur Montage am bzw. im Maschinenelement ausgebildet. Das Fenster schließt einen Innenraum des Sensorgehäuses ab. Im Sensorgehäuse befindet sich ein Sender zum Aussenden elektromagnetischer Strahlung durch das Fenster nach außerhalb des Sensorgehäuses, nämlich auf das zu untersuchende Schmiermittel. Das Fenster muss zumindest für diese elektromagnetische Strahlung transparent sein. Im Sensorgehäuse befindet sich weiterhin ein Empfänger zum Empfangen elektromagnetischer Strahlung, welche durch das Fenster tritt. Dabei handelt es sich um diejenige elektromagnetische Strahlung, welche vom Sender gesendet und vom Schmiermittel reflektiert wurde.

Die erfindungsgemäße Sensoreinheit umfasst weiterhin mindestens eine im Sensorgehäuse angeordnete Ansteuerelektronik und Signalaufbereitungselektronik, welche mit dem Empfänger elektrisch verbunden ist. Die Auswertelektronik ist auch mit dem Sender elektrisch verbunden, in einer Funktion als Ansteuerelektronik. Die Ansteuerelektronik und Signalaufbereitungselektronik ist zur Generierung mindestens eines digitalen Sensorsignals ausgebildet. Folglich erfolgt in der Sensoreinheit nicht lediglich eine Aufbereitung, z. B. eine Verstärkung eines analogen Ausgangssignals des Empfängers. Es erfolgt mindestens eine Analog-Digital-Wandlung des analogen Ausgangssignals des Empfängers, jedoch bevorzugt auch eine Auswertung, sodass das digitale Sensorsignal bereits eine gemessene Eigenschaft des Schmiermittels repräsentiert. Die Ansteuerelektronik und Signalaufbereitungselektronik übernimmt somit auch Funktionen zur Ansteuerung und Signalaufbereitung.

Die erfindungsgemäße Sensoreinheit umfasst weiterhin einen elektrischen Anschluss, welcher durch das Sensorgehäuse hindurchgeführt ist und mit der Ansteuerelektronik und Signalaufbereitungselektronik im Sensorgehäuse elektrisch verbunden ist. Folglich kann das digitale Sensorsignal über den elektrischen Anschluss von außerhalb des Sensorgehäuses abgegriffen werden. Über den elektrischen Anschluss erfolgt bevorzugt auch eine Spannungsversorgung der Ansteuerelektronik und Signalaufbereitungselektronik und des Senders.

Ein besonderer Vorteil der erfindungsgemäßen Sensoreinheit besteht darin, dass sie keine separate Ansteuerelektronik und Signalaufbereitungselektronik, die ausschließlich für einen Betrieb als beabstandete Komponente der Sensoreinheit ausgebildet ist, in einem weiteren Gehäuse erfordert. Folglich umfasst die erfindungsgemäße Sensoreinheit bevorzugt keine solche separate Ansteuerelektronik und Signalaufbereitungselektronik. Vielmehr umfasst die erfindungsgemäße Sensoreinheit bevorzugt ein einziges Gehäuse.

Bei bevorzugten Ausführungsformen der erfindungsgemäßen Sensoreinheit ist die durch den Sender aussendbare elektromagnetische Strahlung durch infrarote Strahlung gebildet. Folglich ist der Empfänger zum Empfangen infraroter Strahlung ausgebildet. Das Fenster ist zumindest für infrarote Strahlung transparent. Die infrarote Strahlung ist besonders gut zur Bestimmung von Eigenschaften eines Schmiermittels geeignet. Die infrarote Strahlung liegt bevorzugt im nahen und/oder mittleren IR-Bereich. Grundsätzlich kann auch andere elektromagnetische Strahlung, wie zum Beispiel sichtbares Licht verwendet werden.

Die Ansteuerelektronik und Signalaufbereitungselektronik umfasst mindestens einen A/D-Wandler zur Wandlung eines analogen Ausgangssignals des Empfängers in das digitale Sensorsignal.

Bei bevorzugten Ausführungsformen der erfindungsgemäßen Sensoreinheit sind der Empfänger und die Ansteuerelektronik und Signalaufbereitungselektronik dazu konfiguriert, elektromagnetische Strahlung im Bereich des Infraroten, bevorzugt im Bereich des nahen und/oder mittleren IR zu erfassen und spektral zu analysieren. Durch die Erfassung und spektrale Auswertung der vom Schmiermittel reflektierten IR-Strahlung können mehrere Eigenschaften des Schmiermittels bestimmt werden.

Hierzu sind der Empfänger und die Ansteuerelektronik und Signalaufbereitungselektronik bevorzugt dazu konfiguriert, elektromagnetische Strahlung im Bereich der Kombinationsmethoden der C-H-Schwingungen zu erfassen und zu analysieren. Im Bereich der C-H-Kombinationsmoden lassen sich einzelne Absorptionslinien erkennen, die Rückschlüsse auf die chemische Zusammensetzung des Schmiermittels zulassen.

Der Sender ist elektrisch mit der Ansteuerelektronik und Signalaufbereitungselektronik verbunden, wobei die Ansteuerelektronik und Signalaufbereitungselektronik bevorzugt zur Ansteuerung des Senders mit unterschiedlichen spektralen Eigenschaften der aussendbaren elektromagnetischen Strahlung konfiguriert ist. Die Ansteuerung des Senders zur Ausstrahlung elektromagnetischer Strahlung mit unterschiedlichen spektralen Eigenschaften unter Berücksichtigung der am Empfänger gemessenen Strahlung erlaubt eine spektrale Analyse der Reflexionseigenschaften des Schmiermittels.

Bei bevorzugten Ausführungsformen der erfindungsgemäßen Sensoreinheit ist die Ansteuerelektronik und Signalaufbereitungselektronik dazu konfiguriert, ausgehend von der Ansteuerung des Senders und ausgehend von einem Ausgangsignal des Empfängers mindestens ein Signal zur Repräsentation einer Eigenschaft des Schmiermittels zu bestimmen, welches das digitale Sensorsignal bzw. eines der digitalen Sensorsignale bildet.

Die durch eine spektrale Analyse zu bestimmenden Eigenschaften des Schmiermittels sind bevorzugt durch einen Zustand des Schmiermittels, insbesondere durch einen Wassergehalt des Schmiermittels, durch eine Trübung des Schmiermittels, durch eine Alterung des Schmiermittels und/oder durch eine Verwendbarkeit des Schmiermittels gebildet. Diese Eigenschaften werden bevorzugt quantitativ erfasst. Folglich repräsentiert das digitale Sensorsignal bzw. jeweils eines der digitalen Sensorsignale bevorzugt einen Wassergehalt des Schmiermittels, eine Trübung des Schmiermittels, eine Alterung des Schmiermittels oder eine Verwendbarkeit des Schmiermittels.

Die erfindungsgemäße Sensoreinheit umfasst bevorzugt weiterhin einen im Sensorgehäuse angeordneten Temperatursensor zum Messen einer Temperatur des Schmiermittels. Dabei ist eines der digitalen Sensorsignale bevorzugt aus einem Ausgangssignal des Temperatursensors gebildet. Im Übrigen kann die gemessene Temperatur ergänzend zur Bestimmung der spektralen Reflexionseigenschaften des Schmiermittels herangezogen werden. Hierfür ist die Ansteuerelektronik und Signalaufbereitungselektronik bevorzugt dazu konfiguriert, ausgehend von der Ansteuerung des Senders und ausgehend von einem Ausgangsignal des Empfängers sowie ausgehend von einem Ausgangssignal des Temperatursensors mindestens ein Signal zur Repräsentation einer Eigenschaft des Schmiermittels zu bestimmen, welches das digitale Sensorsignal bzw. eines der digitalen Sensorsignale bildet.

Der Sender ist bevorzugt durch eine Diode, insbesondere eine LED gebildet.

Der elektrische Anschluss ist bevorzugt durch ein Kabel gebildet. Das Kabel ist aus dem Sensorgehäuse herausgeführt und dient dem elektrischen Anschluss der Sensoreinheit.

Ein besonderer Vorteil der erfindungsgemäßen Sensoreinheit besteht darin, dass die Ausgangssignale der Sensoreinheit ausgehend von der am Schmiermittel befindlichen Sensoreinheit über längere Wegstrecken übertragen werden können, da es sich um digitale Signale handelt. Die die aus dem Stand der Technik bekannten Sensorköpfe weisen ein analoges Ausgangssignal auf, welches nur über kurze Strecken von weniger als ein Meter mit ausreichendem Störabstand übertragbar ist. Das Kabel der erfindungsgemäßen Sensoreinheit ist bevorzugt länger als ein Meter.

Der elektrische Anschluss umfasst bevorzugt genau einen zur Datenübertragung ausgebildeten elektrischen Pol, z. B. in Form einer Ader eines Kabels, über welchen die digitalen Sensorsignale sequentiell übertragbar sind. Dieser Pol kann zum Beispiel als ein Ein-Draht-Bus ausgelegt sein. Die digitalen Sensorsignale können beispielsweise auch als PWM-Signal übertragen werden. Auf zwei weiteren Polen können ein Massepotenzial und eine Versorgungsspannung liegen.

Die erfindungsgemäße Sensoreinheit umfasst bevorzugt weiterhin einen Referenzempfänger, wobei der Referenzempfänger und der Empfänger auf zueinander beabstandeten Flächen angeordnet sind. Diese Flächen sind in Richtung der vom Sender aussendbaren Strahlung zueinander beabstandet und bevorzugt parallel zueinander ausgerichtet. Dabei umfasst die erfindungsgemäße Sensoreinheit bevorzugt weiterhin eine Spiegelfläche, an der die vom Sender aussendbare elektromagnetische Strahlung reflektierbar und dem Referenzempfänger zuführbar ist. Der Referenzempfänger empfängt also die vom Sender ausgesendete elektromagnetische Strahlung, ohne dass diese eine Reflexion am Schmiermittel durchlaufen hat. Durch einen Vergleich der mit dem Referenzempfänger empfangenen elektromagnetischen Strahlung mit der mit dem Empfänger empfangenen elektromagnetischen Strahlung lässt sich die spektrale Beeinflussung durch die Reflexion am Schmiermittel unter Ausschluss von Störgrößen bestimmen. Der Sender und der Referenzempfänger sind bevorzugt auf einem Träger angeordnet, während der Empfänger und die Spiegelfläche auf entgegengesetzten flachen Seiten eines Brückenelementes angeordnet sind. Dabei ist die Spiegelfläche dem Träger zugewandt und der Empfänger dem Träger abgewandt. Der Träger und das Brückenelement sind beabstandet zueinander angeordnet, wobei diese Beabstandung in Richtung der vom Sender aussendbaren Strahlung ausgebildet ist.

Alternativ kann der Referenzempfänger gegenüber einem den Sender gleichenden weiteren Sender angeordnet sein.

Der Sender, der Empfänger und ggf. der Referenzempfänger sind bevorzugt als DIES ausgebildet.

Das Sensorgehäuse weist bevorzugt eine zylindrische Grundform auf, beispielsweise eine stiftartige Grundform. Das Fenster ist dabei an einem axialen Ende der zylindrischen Grundform angeordnet. Am anderen axialen Ende kann sich ein Befestigungselement befinden, beispielsweise ein Außengewinde mit aufgeschraubter Mutter und mit einem Anschlag, jedoch ist bevorzugt kein solches Befestigungselement vorhanden, wobei die Befestigung der Sensoreinheit durch die zylindrische Grundform des Sensorgehäuses formschlüssig oder kraftschlüssig erfolgt, beispielsweise unter Verwendung einer Spannschraube.

Das Schmiermittel ist bevorzugt durch ein Schmierfett gebildet.

Das erfindungsgemäße Maschinenelement weist bewegliche Komponenten auf, zwischen denen ein Schmiermittel angeordnet ist. Das Schmiermittel dient dazu, die Reibung zwischen den beweglichen Komponenten zu mindern. Das Maschinenelement umfasst die erfindungsgemäße Sensoreinheit, wobei das Fenster der Sensoreinheit in das Schmiermittel ragt. Die Sensoreinheit ist bevorzugt im oder am Maschinenelement befestigt.

Bevorzugte Ausführungsformen des erfindungsgemäßen Maschinenelementes umfassen bevorzugte Ausführungsformen der erfindungsgemäßen Sensoreinheit.

Das Maschinenelement ist bevorzugt durch ein Wälzlager gebildet, jedoch kommen auch andere Arten von Maschinenelementen infrage.

Die erfindungsgemäße Maschinenanordnung umfasst das erfindungsgemäße Maschinenelement und eine frei programmierbare Recheneinheit. Der elektrische Anschluss der Sensoreinheit ist mit der frei programmierbaren Recheneinheit elektrisch verbunden, sodass das digitale Sensorsignal in der frei programmierbaren Recheneinheit verarbeitet werden kann, beispielsweise um Eigenschaften des zu untersuchenden Schmiermittels zu bestimmen. Daher ist auf die frei programmierbare Recheneinheit bevorzugt ein Programm zur Auswertung des digitalen Sensorsignals geladen.

Ein besonderer Vorteil der erfindungsgemäßen Sensoreinheit besteht nämlich darin, dass sie keine separate Ansteuerelektronik und Signalaufbereitungselektronik, die ausschließlich für einen Betrieb als beabstandete Komponente der Sensoreinheit ausgebildet ist, in einem weiteren Gehäuse erfordert. Stattdessen wird die erfindungsgemäße Sensoreinheit bevorzugt in Kombination mit einer frei programmierbaren Recheneinheit betrieben, auf welcher ein entsprechendes Programm zur Auswertung des digitalen Sensorsignals geladen ist.

Bevorzugte Ausführungsformen der erfindungsgemäßen Maschinenanordnung umfassen bevorzugte Ausführungsformen des erfindungsgemäßen Maschinenelementes.

Weitere Vorteile, Einzelheiten und Weiterbildungen der Erfindung ergeben sich aus der nachfolgenden Beschreibung einer bevorzugten Ausführungsform der erfindungsgemäßen Sensoreinheit, unter Bezugnahme auf die Zeichnung.

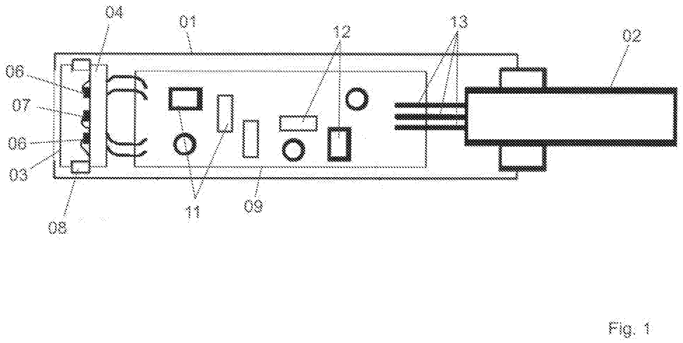

Die einzige Fig. 1 zeigt eine bevorzugte Ausführungsform der erfindungsgemäßen Sensoreinheit in einer schematischen Darstellung. Die Sensoreinheit umfasst zunächst ein zylinderförmiges Sensorgehäuse 01, aus welchem ein dreipoliges Kabel 02 zum elektrischen Anschluss der Sensoreinheit herausgeführt ist. An dem anderen axialen Ende der Zylinderform des Sensorgehäuses 01 bildet eine Saphirscheibe 03 einen Teil des Sensorgehäuses 01.

Die gezeigte Sensoreinheit dient zum Bestimmen der Eigenschaften von Schmierfett, welches sich in Gebrauch befindet, z. B. in einem laufenden Wälzlager. Hierzu ist die Sensoreinheit im Wälzlager (nicht gezeigt) befestigt, wobei die Saphirscheibe 03 in das im Wälzlager angeordnete Schmierfett ragt, sodass sich das Schmierfett auf der Saphirscheibe 03 befindet. Die Saphirscheibe 03 bildet dabei ein Fenster des Sensorgehäuses 01.

Im Inneren des Sensorgehäuses 01 befindet sich hinter Saphirscheibe 03 eine Messanordnung 04 mit Infrarot-Sendern 06 und mit einem Infrarot-Empfänger 07. Die Messanordnung 04 ist durch Abstandshalter 08 von der Saphirscheibe 03 beabstandet angeordnet. Mithilfe der Infrarot-Sender 06 kann Infrarot-Strahlung auf das an der Saphirscheibe 03 befindliche Schmierfett (nicht gezeigt) gestrahlt werden, welche vom Schmierfett zurück zu dem Infrarot-Empfänger 07 reflektiert wird. Durch die Reflexion am Schmierfett ändert sich die spektrale Zusammensetzung der Infrarot-Strahlung, wobei die auftretenden Änderungen Rückschlüsse auf die Eigenschaften des Schmierfettes ermöglichen.

Im Inneren des Sensorgehäuses 01 befindet sich weiterhin eine Platine 09, auf welcher einer Ansteuerelektronik 11 und einer Signalaufbereitungselektronik 12 angeordnet sind. Die Ansteuerelektronik 11 und die Signalaufbereitungselektronik 12 sind elektrisch mit den Infrarot-Sendern 06 und mit dem Infrarot-Empfänger 07 verbunden. Eine gezielte Ansteuerung der Infrarot-Sender 06 ermöglicht eine spektrale Auswertung der vom Schmierfett reflektierten Infrarot-Strahlung.

Erfindungsgemäß ist die Signalaufbereitungselektronik 12 gemeinsam mit der Messanordnung 04 in dem Sensorgehäuse 01 angeordnet. Es bedarf keiner weiteren besonderen Ansteuerelektronik und Signalaufbereitungselektronik in einem separaten Gehäuse. Stattdessen kann die Sensoreinheit über das Kabel 02 direkt beispielsweise an einen frei programmierbaren Rechner angeschlossen werden, welcher auch weitere Steuerungs-, Regelungs- und/oder Überwachungsfunktionen der Maschine realisiert. In einem einfachen Fall kann die Sensoreinheit auch direkt an einen Signalgeber angeschlossen werden, welcher dem Bediener ein Warnsignal gibt, sobald das Schmierfett zu wechseln ist.

Die Signalaufbereitungselektronik 12 ist gemeinsam mit der Ansteuerelektronik 11 dazu ausgebildet, aus dem Signal des Infrarot-Empfängers 07 unter Berücksichtigung der Ansteuerung der Infrarot-Sender 06 ein digitales Sensorsignal zu generieren, welches eine oder mehrere der ermittelten Eigenschaften des Schmierfettes repräsentiert. Das Sensorsignal wird beispielsweise als ein Ein-Draht-Bussignal oder als ein PWM-kodiertes Signal über eine von drei Adern 13 des Kabels 02 übertragen. Die beiden anderen Adern 13 des Kabels 02 sind für ein Versorgungsspannung und ein Massepotenzial vorgesehen.

### Bezugszeichenliste

- 01: Sensorgehäuse
- 02: Kabel
- 03: Saphirscheibe
- 04: Messanordnung
- 05: -
- 06: Infrarot-Sender
- 07: Infrarot-Empfänger
- 08: Abstandshalter
- 09: Platine
- 10: -
- 11: Ansteuerelektronik
- 12: Signalaufbereitungselektronik
- 13: Adern

## Patentansprüche

1. Sensoreinheit zum Bestimmen von Eigenschaften eines Schmiermittels, folgende Komponenten umfassend:
- ein Sensorgehäuse (01) mit einem Fenster (03), wobei das Fenster (03) auf das Schmiermittel ausrichtbar ist;
- einen im Sensorgehäuse (01) angeordneten Sender (06) zum Aussenden elektromagnetischer Strahlung durch das Fenster (03);
- einen im Sensorgehäuse (01) angeordneten Empfänger (07) zum Empfangen elektromagnetischer Strahlung durch das Fenster (03);
- eine im Sensorgehäuse (01) angeordnete Ansteuerelektronik (11) und Signalaufbereitungselektronik (12), wobei die Ansteuerelektronik (11) und die Signalaufbereitungselektronik (12) mit dem Sender (06) und mit dem Empfänger (07) elektrisch verbunden sind und zur Generierung mindestens eines digitalen Sensorsignals ausgebildet sind; und
- einen elektrischen Anschluss (02), welcher durch das Sensorgehäuse (01) hindurchgeführt ist und mit der Ansteuerelektronik (11) und der Signalaufbereitungselektronik (12) elektrisch verbunden ist, und wobei der elektrische Anschluss (02) an eine frei programmierbare Recheneinheit verbindbar ist; **dadurch gekennzeichnet, dass** die Ansteuerelektronik (11) und die Signalaufbereitungselektronik (12) mindestens einen A/D-Wandler zur Wandlung eines analogen Ausgangssignals des Empfängers (07) in das digitale Sensorsignal umfasst.

2. Sensoreinheit nach Anspruch 1, **dadurch gekennzeichnet, dass** die durch den Sender (06) aussendbare elektromagnetische Strahlung durch infrarote Strahlung gebildet ist.

3. Sensoreinheit nach Anspruch 1 oder 2, **dadurch gekennzeichnet, dass** der Empfänger (07) und die Ansteuerelektronik (11) und die Signalaufbereitungsetektronik (12) dazu konfiguriert sind, elektromagnetische Strahlung im Bereich des Infraroten zu erfassen und spektral zu analysieren.

4. Sensoreinheit nach einem der Ansprüche 1 bis 3, **dadurch gekennzeichnet, dass** die Ansteuerelektronik (11) und die Signalaufbereitungselektronik (12) dazu konfiguriert sind, ausgehend von der Ansteuerung des Senders (06) und ausgehend von dem analogen Ausgangsignal des Empfängers (07) mindestens ein Signal zur Repräsentation einer Eigenschaft des Schmiermittels zu bestimmen, welches das digitale Sensorsignal bildet.

5. Sensoreinheit nach einem der Ansprüche 1 bis 4, **dadurch gekennzeichnet, dass** das digitale Sensorsignal einen Zustand des Schmiermittels repräsentiert.

6. Sensoreinheit nach einem der Ansprüche 1 bis 5, **dadurch gekennzeichnet, dass** das digitale Sensorsignal eine Verwendbarkeit des Schmiermittels repräsentiert.

7. Sensoreinheit nach einem der Ansprüche 1 bis 6, **dadurch gekennzeichnet, dass** sie weiterhin einen im Sensorgehäuse (01) angeordneten Temperatursensor zum Messen einer Temperatur des Schmiermittels umfasst.

8. Maschinenelement mit beweglichen Komponenten, zwischen denen ein Schmiermittel angeordnet ist, **dadurch gekennzeichnet, dass** es weiterhin eine Sensoreinheit nach einem der Ansprüche 1 bis 7 umfasst, wobei das Fenster (03) der Sensoreinheit in das Schmiermittel ragt.

9. Maschinenanordnung, umfassend ein Maschinenelement nach Anspruch 8 und eine frei programmierbare Recheneinheit, wobei der elektrische Anschluss (02) der Sensoreinheit mit der frei programmierbaren Recheneinheit elektrisch verbunden ist.

## Claims

1. Sensor unit for determining properties of a lubricant, comprising the following components:
- a sensor housing (01) having a window (03), the window (03) being able to be aligned with the lubricant;
- a transmitter (06) which is arranged in the sensor housing (01) and is intended to emit electromagnetic radiation through the window (03) ;
- a receiver (07) which is arranged in the sensor housing (01) and is intended to receive electromagnetic radiation through the window (03);
- control electronics (11) and signal conditioning electronics (12) which are arranged in the sensor housing (01), the control electronics (11) and the signal conditioning electronics (12) being electrically connected to the transmitter (06) and to the receiver (07) and being designed to generate at least one digital sensor signal; and
- an electrical connection (02) which is guided through the sensor housing (01) and is electrically connected to the control electronics (11) and to the signal conditioning electronics (12), and the electrical connection (02) being able to be connected to a freely programmable computing unit;
**characterized in that**
the control electronics (11) and the signal conditioning electronics (12) comprise at least one A/D converter for converting an analogue output signal from the receiver (07) into the digital sensor signal.

2. Sensor unit according to Claim 1, **characterized in that** the electromagnetic radiation which can be emitted by the transmitter (06) is formed by infrared radiation.

3. Sensor unit according to Claim 1 or 2, **characterized in that** the receiver (07) and the control electronics (11) and the signal conditioning electronics (12) are configured to capture and spectrally analyse electromagnetic radiation in the infrared range.

4. Sensor unit according to one of Claims 1 to 3, **characterized in that** the control electronics (11) and the signal conditioning electronics (12) are configured to determine at least one signal for representing a property of the lubricant on the basis of the control of the transmitter (06) and on the basis of the analogue output signal from the receiver (07), which signal forms the digital sensor signal.

5. Sensor unit according to one of Claims 1 to 4, **characterized in that** the digital sensor signal represents a state of the lubricant.

6. Sensor unit according to one of Claims 1 to 5, **characterized in that** the digital sensor signal represents usability of the lubricant.

7. Sensor unit according to one of Claims 1 to 6, **characterized in that** it also comprises a temperature sensor which is arranged in the sensor housing (01) and is intended to measure a temperature of the lubricant.

8. Machine element having movable components between which a lubricant is arranged, **characterized in that** it also comprises a sensor unit according to one of Claims 1 to 7, the window (03) of the sensor unit projecting into the lubricant.

9. Machine arrangement comprising a machine element according to Claim 8 and a freely programmable computing unit, the electrical connection (02) of the sensor unit being electrically connected to the freely programmable computing unit.

## Revendications

1. Unité de capteur servant à déterminer les propriétés d'un lubrifiant, l'unité comportant les composants suivantes :
un boîtier (01) de capteur présentant une fenêtre (03), la fenêtre (03) pouvant être orientée vers le lubrifiant,
un émetteur (06) disposé dans le boîtier (01) de capteur et émettant un rayonnement électromagnétique à travers la fenêtre (03),
un récepteur (07) disposé dans le boîtier (01) du capteur et recevant le rayonnement électromagnétique ayant traversé la fenêtre (03),
une électronique de commande (11) disposée dans le boîtier (01) du capteur et une électronique (12) de traitement du signal, l'électronique de commande (11) et l'électronique (12) de traitement du signal étant raccordées électriquement à l'émetteur (06) et au récepteur (07) et étant configurées pour produire au moins un signal numérique de capteur et
un raccordement électrique (02) qui traverse le boîtier (01) du capteur et qui est raccordé électriquement à l'électronique de commande (11) et à l'électronique (12) de traitement du signal, le raccordement électrique (02) pouvant être raccordé à une unité de calcul librement programmable, **caractérisée en ce que**
l'électronique de commande (11) et l'électronique (12) de traitement du signal comportent au moins un convertisseur analogique-numérique qui convertit un signal analogique de sortie du récepteur (07) en le signal numérique de capteur.

2. Unité de capteur selon la revendication 1, **caractérisée en ce que** le rayonnement électromagnétique qui peut être émis par l'émetteur (06) est formé d'un rayonnement infrarouge.

3. Unité de capteur selon l'une des revendications 1 ou 2, **caractérisée en ce que** le récepteur (07), l'électronique de commande (11) et l'électronique (12) de traitement du signal sont configurés pour saisir le rayonnement électromagnétique dans la plage de l'infrarouge et d'en faire l'analyse spectrale.

4. Unité de capteur selon l'une des revendications 1 à 3, **caractérisée en ce que** l'électronique de commande (11) et l'électronique (12) de traitement du signal sont configurées pour déterminer à partir de la commande de l'émetteur (06) et du signal analogique de sortie du récepteur (07) au moins un signal qui représente une propriété du lubrifiant, ce signal formant le signal numérique du capteur.

5. Unité de capteur selon l'une des revendications 1 à 4, **caractérisé en ce que** le signal numérique de capteur représente l'état du lubrifiant.

6. Unité de capteur selon l'une des revendications 1 à 5, **caractérisé en ce que** le signal numérique du capteur représente la possibilité d'encore utiliser le lubrifiant.

7. Unité de capteur selon l'une des revendications 1 à 6, **caractérisé en ce qu'**il comporte en outre un capteur de température disposé dans le boîtier (01) du capteur et qui mesure la température du lubrifiant.

8. Elément de machine présentant des composants mobiles entre lesquels est disposé un lubrifiant, **caractérisé en ce qu'**il comporte en outre une unité de capteur selon l'une des revendications 1 à 7, la fenêtre (03) de l'unité de capteur pénétrant dans le lubrifiant.

9. Ensemble de machine comprenant un élément de machine selon la revendication 8 et une unité de calcul librement programmable, le raccordement électrique (02) de l'unité de capteur étant raccordé électriquement à l'unité de calcul librement programmable.
